(19) **Europäisches Patentamt / European Patent Office / Office européen des brevets**

(11) **EP 4 682 235 A1**

(12) # EUROPEAN PATENT APPLICATION

(43) Date of publication:
**21.01.2026 Bulletin 2026/04**

(21) Application number: **24188554.0**

(22) Date of filing: **15.07.2024**

(51) International Patent Classification (IPC):
**C11D 3/37** $^{(2006.01)}$  **C11D 3/38** $^{(2006.01)}$
**C11D 17/04** $^{(2006.01)}$

(52) Cooperative Patent Classification (CPC):
**C11D 3/381; C11D 3/3753; C11D 17/042;**
C12R 2001/07

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **The Procter & Gamble Company
Cincinnati, OH 45202 (US)**

(72) Inventors:
- **COURCHAY, Florence Catherine
  1853 Strombeek-Bever (BE)**
- **LANT, Neil Joseph
  Newcastle upon Tyne, NE12 9TS (GB)**
- **LATIMER, Katherine Esther
  Newcastle upon Tyne, NE12 9TS (GB)**

(74) Representative: **P&G Patent Belgium UK
N.V. Procter & Gamble Services Company S.A.
Temselaan 100
1853 Strombeek-Bever (BE)**

(54) **WATER-SOLUBLE UNIT DOSE ARTICLE**

(57) A water-soluble unit-dose detergent article comprising a water-soluble film and a detergent composition, wherein the water-soluble film comprises bacterial spores.

**Description**

FIELD OF THE INVENTION

Water-soluble unit dose detergent article and a method of making and using the article of the invention.

BACKGROUND OF THE INVENTION

Fabric malodor can become a problem with the current trend of using lower washing temperatures and shorter cycles. The obj ect of the present invention is to provide a detergent article that provides improved malodor removal, even when used at low water temperatures and in short cycles. The detergent article should be stable during its lifetime, i.e. during transport, in store and in the consumer home.

SUMMARY OF THE INVENTION

According to a first aspect of the present invention, there is provided a water-soluble unit dose article. The water-soluble unit dose article comprises a water-soluble film and a detergent composition. The detergent composition is enveloped by the water-soluble film. The detergent composition comprises a surfactant and the water-soluble film comprises bacterial spores.

According to a second aspect of the present invention, there is provided a process for making the water-soluble unit dose detergent article of the invention, comprising the steps of:

a) forming a film comprising bacterial spores; and

b) encapsulating the detergent composition in the water-soluble film.

According to a third aspect of the present invention, there is provided a method of laundering a fabric with the water-soluble unit dose article of the invention.

The water-soluble unit dose article is stable during its lifetime and provides good fabric malodour removal and/or fabric malodour prevention.

DETAILED DESCRIPTION OF THE INVENTION

As used herein, the articles including "the," "a" and "an" when used in a claim or in the specification, are understood to mean one or more of what is claimed or described.

As used herein, the terms "include," "includes" and "including" are meant to be nonlimiting.

All percentages, ratios and proportions used herein are by weight percent of the composition, unless otherwise specified. All average values are calculated "by weight" of the composition, unless otherwise expressly indicated.

All measurements are performed at 25°C unless otherwise specified.

Unless otherwise noted, all component or composition levels are in reference to the active portion of that component or composition, and are exclusive of impurities, for example, residual solvents or by-products, which may be present in commercially available sources of such components or compositions.

Unit-dose article

The present invention relates to a unit-dose article. The unit-dose article comprises a water-soluble film which encompasses an inner volume enclosed by the water-soluble film. The inner volume houses the liquid composition of the invention.

Multiple compartments, also referred to herein as multi-compartment, unit-dose articles comprise two or more compartments.

The water-soluble film is sealed such that the composition does not leak out of the compartments during storage. However, upon addition of the water-soluble unit-dose article to water, the water-soluble film dissolves and releases the contents of the internal compartment into water.

Each compartment should be understood as meaning a closed internal space within the unit-dose article, which holds a composition. The unit-dose article is manufactured such that the water-soluble film completely surrounds a composition and in doing so defines the compartment in which the composition resides. The film is described in more detail below.

The unit-dose article can comprise one or more compartments, two or even at least three compartments, or even at least four compartments, in which preferably at least two compartments are present in a superposed position, i.e. one positioned on top of the other. The unit-dose article may further comprise compartments positioned in a side-by-side orientation, i.e. one orientated next to one another, or even be orientated in a 'tyre and rim' arrangement, i.e. a first compartment is positioned next to a second compartment, but the first compartment at least partially surrounds the second compartment, but does not completely enclose the second compartment, or alternatively, one compartment may be completely enclosed within another compartment.

One of the compartments may be smaller than the other compartment. Wherein the unit-dose article comprises at least three compartments, two of the compartments may be smaller than the third compartment, and preferably the smaller compartments are superposed on the larger compartment. Wherein the unit-dose article comprises at least four compartments, three of the compartments may be smaller than the fourth compartment, and preferably the smaller compartments are superposed on the larger compartment. The superposed compartments preferably are orientated side-by-side.

The water-soluble film comprises an inner surface which is in contact with a composition and an outer surface which is oriented away from the composition, towards the outside environment.

Preferably the water-soluble unit-dose article comprises one larger compartment with at least one, preferably at least two, or even at least three smaller compartments superposed thereon. Preferably the first composition is housed in a smaller compartment (first compartment) and the second composition is housed in a larger compartment (second compartment).

The outer contouring seal area includes or preferably consists of a flange area. A flange area is arranged around the perimeter of the unit-dose article, and the flange comprises sealed film from two, three, or more water-soluble films. In other words, the flange area protrudes out from the unit-dose article and comprises sealed film. By 'seal area' we herein mean both the inner seal area as defined as the areas of film sealed together to define the individual compartments without the presence of a flange as well as the outer seal area defining a flange of the water-soluble unit-dose article. Herein the flange excludes the inner seal areas. Preferably, the flange comprises sealed film from at least a first water-soluble film and a second water-soluble film and a third water-soluble film if present. The inner seal area can be created by sealing two water-soluble films together to create physically separated individual compartments or can be created by sealing at least three films together to create physically separated individual compartments. Preferably the inner seal is created by sealing solely two water-soluble films together.

Water-soluble film

The film of the unit-dose article of the present invention is soluble or dispersible in water. The water-soluble film preferably has a thickness prior to deformation of from 20 to 150 micron, preferably 35 to 125 micron, even more preferably 50 to 110 micron, most preferably about 76 micron.

Preferably, the film has a water-solubility of at least 50%, preferably at least 75% or even at least 95%, as measured by the method set out here after using a glass-filter with a maximum pore size of 20 microns:
5 grams $\pm$ 0.1 gram of film material is added in a pre-weighed 3L beaker and 2L * 5ml of distilled water is added. This is stirred vigorously on a magnetic stirrer, Labline model No. 1250 or equivalent and 5 cm magnetic stirrer, set at 600 rpm, for 30 minutes at 30oC. Then, the mixture is filtered through a folded qualitative sintered-glass filter with a pore size as defined above (max. 20 micron). The water is dried off from the collected filtrate by any conventional method, and the weight of the remaining material is determined (which is the dissolved or dispersed fraction). Then, the percentage solubility or dispersability can be calculated.

Preferred film materials are preferably polymeric materials. The film material can, for example, be obtained by casting, blow-moulding, extrusion or blown extrusion of the polymeric material, as known in the art.

The water-soluble film comprises polyvinyl alcohol polymer wherein the polyvinyl alcohol polymer comprises a polyvinyl alcohol homopolymer, an anionic polyvinyl alcohol copolymer, or a blend thereof, preferably wherein the anionic polyvinylalcohol copolymers are selected from sulphonated and carboxylated anionic polyvinylalcohol copolymers especially carboxylated anionic polyvinylalcohol copolymers. Most preferably the water-soluble film comprises a blend of polyvinyl alcohol homopolymers, a blend of a polyvinylalcohol homopolymer and a carboxylated anionic polyvinyl-lalcohol copolymer, or alternatively, the polyvinylalcohol consists of an anionic polyvinyl alcohol copolymer, most preferably a carboxylated anionic polyvinylalcohol copolymer, or alternatively the polyvinylalcohol comprises of a polyvinylalcohol homopolymer. When the polyvinylalcohol in the water-soluble film is a blend of a polyvinylalcohol homopolymer and a carboxylated anionic polyvinylalcohol copolymer, the homopolymer and the anionic copolymer are present in a relative weight ratio of 90/10 to 10/90, preferably 80/20 to 20/80, more preferably 70/30 to 50/50. General classes of anionic monomer units which can be used for the anionic polyvinyl alcohol co-polymer include the vinyl polymerization units corresponding to monocarboxylic acid vinyl monomers, their esters and anhydrides, dicarboxylic monomers having a polymerizable double bond, their esters and anhydrides, vinyl sulfonic acid monomers, and alkali metal salts of any of the foregoing. Examples of suitable anionic monomer units include the vinyl polymerization units corresponding to vinyl anionic monomers including vinyl acetic acid, maleic acid, monoalkyl maleate, dialkyl maleate, monomethyl maleate, dimethyl maleate, maleic any hydride, fumaric acid, monoalkyl fumarate, dialkyl fumarate, monomethyl fumarate, dimethyl fumarate, fumaric anyhydride, itaconic acid, monomethyl itaconate, dimethyl itaconate, itaconic anhydride, vinyl sulfonic acid, allyl sulfonic acid, ethylene sulfonic acid, 2-acrylamido-1-methylpropanesulfonic acid, 2-acrylamido-2-methylpropanesulfonic acid, 2-methylacrylamido-2-methylpropanesulfonic acid, 2-sufoethyl acrylate, alkali metal salts of the foregoing (e.g., sodium, potassium, or other alkali metal salts), esters of the foregoing (e.g., methyl, ethyl, or other C1-C4 or C6 alkyl esters), and combinations thereof (e.g., multiple types of anionic monomers or equivalent forms of the same anionic monomer). The anionic monomer may be one or more acrylamido methylpropanesulfonic acids (e.g., 2-acrylamido-1-methylpropanesulfonic acid, 2-acrylamido-2-methylpropanesulfonic acid, 2-methylacrylamido-2-methylpropanesulfonic acid), alkali metal salts thereof (e.g., sodium salts), and combinations thereof. Preferably, the anionic moiety of the first anionic monomer unit is selected from a sulphonate, a carboxylate, or a mixture thereof, more preferably a carboxylate, most preferably an acrylate, a methacrylate, a maleate, or a mixture thereof. Preferably, the anionic monomer unit is present in the anionic polyvinyl alcohol copolymer in an average amount in a range of between 1 mol.% and 5 mol.% or between 2 mol.% and 5 mol.%.

The polyvinyl alcohol polymer may be present between 50% and 95%, preferably between 55% and 90%, more preferably between 60% and 80% by weight of the water-soluble film.

Without wishing to be bound by theory, the term "homopolymer" generally includes polymers having a single type of monomeric repeating unit (e.g., a polymeric chain comprising or consisting of a single monomeric repeating unit). For the particular case of polyvinyl alcohol, the term "homopolymer" further includes copolymers having a distribution of vinyl alcohol monomer units and optionally vinyl acetate monomer units, depending on the degree of hydrolysis (e.g., a polymeric chain comprising or consisting of vinyl alcohol and vinyl acetate monomer units). In the case of 100% hydrolysis, a polyvinyl alcohol homopolymer can include only vinyl alcohol units. Without wishing to be bound by theory, the term "copolymer" generally includes polymers having two or more types of monomeric repeating units (e.g., a polymeric chain comprising or consisting of two or more different monomeric repeating units, whether as random copolymers, block copolymers, etc.). For the particular case of polyvinyl alcohol, the term "copolymer" (or "polyvinyl alcohol copolymer") further includes copolymers having a distribution of vinyl alcohol monomer units and vinyl acetate monomer units, depending on the degree of hydrolysis, as well as at least one other type of monomeric repeating unit (e.g., a ter- (or higher) polymeric chain comprising or consisting of vinyl alcohol monomer units, vinyl acetate monomer units, and one or more other monomer units, for example anionic monomer units). In the case of 100% hydrolysis, a polyvinyl alcohol copolymer can include a copolymer having vinyl alcohol units and one or more other monomer units, but no vinyl acetate units. Without wishing to be bound by theory, the term "anionic copolymer" includes copolymers having an anionic monomer unit comprising an anionic moiety.

Preferably, the polyvinyl alcohol, and/or in case of polyvinyl alcohol blends the individual polyvinyl alcohol polymers and/or the combined polyvinyl alcohol polymers, have an average viscosity ($\mu 1$) in a range of between 4 mPa.s and 30 mPa.s, preferably between 10mPa.s and 25 mPa.s, measured as a 4% polyvinyl alcohol polymer solution in demineralized water at 20 degrees C. The viscosity of a polyvinyl alcohol polymer is determined by measuring a freshly made solution using a Brookfield LV type viscometer with UL adapter as described in British Standard EN ISO 15023-2:2006 Annex E Brookfield Test method. It is international practice to state the viscosity of 4% aqueous polyvinyl alcohol solutions at 20 °C. It is well known in the art that the viscosity of an aqueous water-soluble polymer solution (polyvinylalcohol or otherwise) is correlated with the weight-average molecular weight of the same polymer, and often the viscosity is used as a proxy for weight-average molecular weight. Thus, the weight-average molecular weight of the polyvinylalcohol can be in a range of 30,000 to 175,000, or 30,000 to 100,000, or 55,000 to 80,000. Preferably, the polyvinyl alcohol, and/or in case of polyvinylalcohol blends the individual polyvinylalcohol polymers, have an average degree of hydrolysis in a range of between 75% and 99%, preferably between 80% and 95%, most preferably between 85% and 95%. A suitable test method to measure the degree of hydrolysis is as according to standard method JIS K6726.

The water-soluble film may comprise a material selected from the group consisting of protein, polysaccharide, and a mixture thereof. The film can comprise casein, pectin, carrageenan, or a mixture thereof.

Preferably, the water-soluble film comprises a non-aqueous plasticizer. Preferably, the non-aqueous plasticizer is selected from polyols, sugar alcohols, and mixtures thereof. Suitable polyols include polyols selected from the group consisting of glycerol, diglycerin, ethylene glycol, diethylene glycol, triethyleneglycol, tetraethylene glycol, polyethylene glycols up to 400 molecular weight, neopentyl glycol, 1,2-propylene glycol, 1,3-propanediol, dipropylene glycol, polypropylene glycol, 2-methyl-1,3-propanediol, trimethylolpropane and polyether polyols, or a mixture thereof. Suitable sugar alcohols include sugar alcohols selected from the group consisting of isomalt, maltitol, sorbitol, xylitol, erythritol, adonitol, dulcitol, pentaerythritol and mannitol, or a mixture thereof. More preferably the non-aqueous plasticizer is selected from glycerol, 1,2-propanediol, dipropylene glycol, 2-methyl-1,3-propanediol, trimethylolpropane, triethyleneglycol, polyethyleneglycol, sorbitol, or a mixture thereof, most preferably selected from glycerol, sorbitol, trimethylolpropane, dipropylene glycol, and mixtures thereof. One particularly suitable plasticizer system includes a blend of glycerol, sorbitol and trimethylol propane. Another particularly suitable plasticizer system includes a blend of glycerin, dipropylene glycol, and sorbitol. Preferably, the film comprises between 5% and 50%, preferably between 10% and 40%, more preferably between 20% and 30% by weight of the film of the non-aqueous plasticizer.

Preferably, the water-soluble film comprises a surfactant. Preferably, the water-soluble film comprises a surfactant in an amount between 0.1% and 2.5%, preferably between 1% and 2% by weight of the water-soluble film. Suitable surfactants can include the nonionic, cationic, anionic and zwitterionic classes. Suitable surfactants include, but are not limited to, polyoxyethylenated polyoxypropylene glycols, alcohol ethoxylates, alkylphenol ethoxylates, tertiary acetylenic glycols and alkanolamides (nonionics), polyoxyethylenated amines, quaternary ammonium salts and quaternized polyoxyethylenated amines (cationics), and amine oxides, N-alkylbetaines and sulfobetaines (zwitterionics). Other suitable surfactants include dioctyl sodium sulfosuccinate, lactylated fatty acid esters of glycerol and propylene glycol, lactylic esters of fatty acids, sodium alkyl sulfates, polysorbate 20, polysorbate 60, polysorbate 65, polysorbate 80, lecithin, acetylated fatty acid esters of glycerol and propylene glycol, and acetylated esters of fatty acids, and combinations thereof.

Preferably the water-soluble film according to the invention comprises lubricants / release agents. Suitable lubricants/release agents can include, but are not limited to, fatty acids and their salts, fatty alcohols, fatty esters, fatty amines, fatty amine acetates and fatty amides. Preferred lubricants/release agents are fatty acids, fatty acid salts, and fatty amine acetates. The amount of lubricant/release agent in the water-soluble film is in a range of from 0.02% to 1.5%, preferably from 0.1% to 1% by weight of the water-soluble film.

Preferably, the water-soluble film comprises fillers, extenders, antiblocking agents, detackifying agents or a mixture thereof. Suitable fillers, extenders, antiblocking agents, detackifying agents or a mixture thereof include, but are not limited to, starches, modified starches, crosslinked polyvinylpyrrolidone, crosslinked cellulose, microcrystalline cellulose, silica, metallic oxides, calcium carbonate, talc and mica. Preferred materials are starches, modified starches and silica. Preferably, the amount of filler, extender, antiblocking agent, detackifying agent or mixture thereof in the water-soluble film is in a range of from 0.1% to 25%, preferably from 1% to 10%, more preferably from 2% to 8%, most preferably from 3% to 5% by weight of the water-soluble film. In the absence of starch, one preferred range for a suitable filler, extender, antiblocking agent, detackifying agent or mixture thereof is from 0.1% to 1%, preferably 4%, more preferably 6%, even more preferably from 1% to 4%, most preferably from 1% to 2.5%, by weight of the water-soluble film.

Preferably the water-soluble film according to the invention has a residual moisture content of at least 4%, more preferably in a range of from 4% to 15%, even more preferably of from 5% to 10% by weight of the water-soluble film as measured by Karl Fischer titration.

Preferred films exhibit good dissolution in cold water, meaning unheated distilled water. Preferably such films exhibit good dissolution at temperatures of 24oC, even more preferably at 10oC. By good dissolution it is meant that the film exhibits water-solubility of at least 50%, preferably at least 75% or even at least 95%, as measured by the method set out here after using a glass-filter with a maximum pore size of 20 microns, described above.

The film may be opaque, transparent or translucent. Preferably, the film is transparent. The film may comprise a printed area.

The area of print may be achieved using standard techniques, such as flexographic printing or inkjet printing.

The film may comprise an aversive agent, for example a bittering agent. Suitable bittering agents include, but are not limited to, naringin, sucrose octaacetate, quinine hydrochloride, denatonium benzoate, or mixtures thereof. Any suitable level of aversive agent may be used in the film. Suitable levels include, but are not limited to, 1 to 5000ppm, or even 100 to 2500ppm, or even 250 to 2000rpm.

The water-soluble film or water-soluble unit-dose article or both may be further coated with powder. The powder preferably has an average particle size of between 0.1 and 20 microns, suitably between 5 and 15 microns. Preferably the powder is selected from talc, zinc oxide, silicas, siloxanes, zeolites, silicic acid, alumina, sodium sulphate, potassium sulphate, calcium carbonate, magnesium carbonate, sodium citrate, sodium tripolyphosphate, potassium citrate, potassium tripolyphosphate, calcium stearate, zinc stearate, magnesium stearate, starch, modified starches, clay, kaolin, gypsum, cyclodextrins or mixtures thereof. The powder is generally applied at a rate of from 0.5 to 10mg/100cm2, preferably not more than 5mg/100cm2, more preferably in the range 1.25. to 2.5mg/100cm2.

Bacterial spores

The bacterial spores are located in the film. Preferably, the film comprises from about $1\times10^3$, preferably from about $1\times10^4$ to about $1\times10^{11}$, preferably $1\times10^{10}$ CFU/g of bacterial spores by weight of the film.

The bacterial spores are fabric-substantive. The bacterial spores of the composition of the invention can germinate on fabrics. The spores can be activated by heat, for example, heat generated during use of the fabric or by the heat provided in the washing machine or in the dryer. The spores can germinate when the fabrics are stored and/or used. Malodor precursors can be used by the bacteria produced by the spores as nutrients promoting germination. Spores can germinate after the fabrics are left in the humid environment.

The spores have the ability to germinate and to form cells after the fabric is subjected to the laundry process. The spores can be delivered in liquid or solid form. Preferably, the spores are in solid form.

Some gram-positive bacteria have a two-stage lifecycle in which growing bacteria under certain conditions such as in response to nutritional deprivation can undergo an elaborate developmental program leading to spores or endospores formation. The bacterial spores are protected by a coat consisting of about 60 different proteins assembled as a biochemically complex structure with intriguing morphological and mechanical properties. The protein coat is considered a static structure that provides rigidity and mainly acting as a sieve to exclude exogenous large toxic molecules, such as lytic enzymes. Spores play critical roles in long term survival of the species because they are highly resistant to extreme environmental conditions. Spores are also capable of remaining metabolically dormant for years. Methods for obtaining bacterial spores from vegetative cells are well known in the field. In some examples, vegetative bacterial cells are grown in liquid medium. Beginning in the late logarithmic growth phase or early stationary growth phase, the bacteria may begin to sporulate. When the bacteria have finished sporulating, the spores may be obtained from the medium, by using centrifugation for example. Various methods may be used to kill or remove any remaining vegetative cells. Various methods may be used to purify the spores from cellular debris and/or other materials or substances. Bacterial spores may be differentiated from vegetative cells using a variety of techniques, like phase-contrast microscopy, automated scanning microscopy, high resolution atomic force microscopy or tolerance to heat, for example.

Because bacterial spores are generally environmentally-tolerant structures that are metabolically inert or dormant, they are readily chosen to be used in commercial microbial products. Despite their ruggedness and extreme longevity, spores can rapidly respond to the presence of small specific molecules known as germinants that signal favorable conditions for breaking dormancy through germination, an initial step in the process of completing the lifecycle by returning to vegetative bacteria. For example, the commercial microbial products may be designed to be dispersed into an environment where the spores encounter the germinants present in the environment to germinate into vegetative cells and perform an intended function. A variety of different bacteria may form spores. For example, some bacteria of the following genera may form spores: *Acetonema, Alkalibacillus, Ammoniphilus, Amphibacillus, Anaerobacter, Anaerospora, Aneurinibacillus, Anoxybacillus, Bacillus, Brevibacillus, Caldanaerobacter , Caloramator, Caminicella, Cerasibacillus, Clostridium, Clostridiisalibacter, Cohnella, Dendrosporobacter, Desulfotomaculum, Desulfosporomusa, Desulfosporosinus, Desulfovirgula, Desulfunispora, Desulfurispora, Filifactor, Filobacillus, Gelria, Geobacillus, Geosporobacter, Gracilibacillus, Halonatronum, Heliobacterium, Heliophilum, Laceyella, Lentibacillus, Lysinibacillus, Mahella, Metabacterium, Moorella, Natroniella, Oceanobacillus, Orenia, Ornithinibacillus, Oxalophagus, Oxobacter, Paenibacillus, Paraliobacillus, Pelospora, Pelotomaculum, Piscibacillus, Planifilum, Pontibacillus, Propionispora, Salinibacillus, Salsuginibacillus, Seinonella, Shimazuella, Sporacetigenium, Sporoanaerobacter, Sporobacter, Sporobacterium, Sporohalobacter, Sporolactobacillus, Sporomusa, Sporosarcina, Sporotalea, Sporotomaculum, Syntrophomonas, Syntrophospora, Tenuibacillus, Tepidibacter, Terribacillus, Thalassobacillus, Thermoacetogenium, Thermoactinomyces, Thermoalkalibacillus, Thermoanaerobacter, Thermoanaeromonas, Thermobacillus, Thermoflavimicrobium, Thermovenabulum, Tuberibacillus, Virgibacillus,* and/ or *Vulcanobacillus.*

Preferably, the bacteria that may form spores are from the family *Bacillaceae,* such as species of the genera *Aeribacillus, Aliibacillus, Alkalibacillus, Alkalicoccus, Alkalihalobacillus, Alkalilactibacillus, Allobacillus, Alteribacillus, Alteribacter,Amphibacillus, Anaerobacillus, Anoxybacillus, Aquibacillus, Aquisalibacillus, Aureibacillus, Bacillus, Caldalkalibacillus, Caldibacillus, Calditerricola, Calidifontibacillus, Camelliibacillus, Cerasibacillus, Compostibacillus, Cytobacillus, Desertibacillus, Domibacillus, Ectobacillus, Evansella, Falsibacillus, Ferdinandcohnia, Fermentibacillus, Fictibacillus, Filobacillus, Geobacillus, Geomicrobium, Gottfriedia, Gracilibacillus, Halalkalibacillus, Halobacillus, Halolactibacillus, Heyndrickxia, Hydrogenibacillus, Lederbergia, Lentibacillus, Litchfieldia, Lottiidibacillus, Margalitia, Marinococcus, Melghiribacillus, Mesobacillus, Metabacillus, Microaerobacter, Natribacillus, Natronobacillus, Neobacillus, Niallia, Oceanobacillus, Ornithinibacillus, Parageobacillus, Paraliobacillus, Paralkalibacillus, Paucisalibacillus, Pelagirhabdus, Peribacillus, Piscibacillus, Polygonibacillus, Pontibacillus, Pradoshia, Priestia, Pseudogracilibacillus, Pueribacillus, Radiobacillus, Robertmurraya, Rossellomorea, Saccharococcus, Salibacterium, Salimicrobium, Salinibacillus, Salipaludibacillus, Salirhabdus, Salisediminibacterium, Saliterribacillus, Salsuginibacillus, Sediminibacillus, Siminovitchia, Sinibacillus, Sinobaca, Streptohalobacillus, Sutcliffiella, Swionibacillus, Tenuibacillus, Tepidibacillus, Terribacillus, Terrilactibacillus, Texcoconibacillus, Thalassobacillus, Thalassorhabdus, Thermolongibacillus, Virgibacillus, Viridibacillu, Vulcanibacillus, Weizmannia.* In various examples, the bacteria may be strains of *Bacillus Bacillus acidicola, Bacillus aeolius, Bacillus aerius, Bacillus aerophilus, Bacillus albus, Bacillus altitudinis, Bacillus alveayuensis, Bacillus amyloliquefaciensex, Bacillus anthracis, Bacillus aquiflavi, Bacillus atrophaeus, Bacillus australimaris, Bacillus badius, Bacillus benzoevorans, Bacillus cabrialesii, Bacillus canaveralius, Bacillus capparidis, Bacillus carboniphilus, Bacillus cereus, Bacillus chungangensis, Bacillus coahuilensis, Bacillus cytotoxicus, Bacillus decisifrondis, Bacillus ectoiniformans, Bacillus enclensis, Bacillus fengqiuensis, Bacillus fungorum, Bacillus glycinifermentans, Bacillus gobiensis, Bacillus halotolerans, Bacillus haynesii, Bacillus horti, Bacillus inaquosorum, Bacillus infantis, Bacillus infernus, Bacillus isabeliae, Bacillus kexueae, Bacillus licheniformis, Bacillus luti, Bacillus manusensis, Bacillus marinisedimentorum, Bacillus mesophilus, Bacillus methanolicus, Bacillus mobilis, Bacillus mojavensis, Bacillus mycoides, Bacillus nakamurai, Bacillus ndiopicus, Bacillus nitratireducens, Bacillus oleivorans, Bacillus pacificus, Bacillus pakistanensis, Bacillus paralicheni-*

formis, Bacillus paramycoides, Bacillus paranthracis, Bacillus pervagus, Bacillus piscicola, Bacillus proteolyticus, Bacillus pseudomycoides, Bacillus pumilus, Bacillus safensis, Bacillus salacetis, Bacillus salinus, Bacillus salitolerans, Bacillus seohaeanensis, Bacillus shivajii, Bacillus siamensis, Bacillus smithii, Bacillus solimangrovi, Bacillus songklensis, Bacillus sonorensis, Bacillus spizizenii, Bacillus spongiae, Bacillus stercoris, Bacillus stratosphericus, Bacillus subtilis, Bacillus swezeyi, Bacillus taeanensis, Bacillus tamaricis, Bacillus tequilensis, Bacillus thermocloacae, Bacillus thermotolerans, Bacillus thuringiensis, Bacillus tianshenii, Bacillus toyonensis, Bacillus tropicus, Bacillus vallismortis, Bacillus velezensis, Bacillus wiedmannii, Bacillus wudalianchiensis, Bacillus xiamenensis, Bacillus xiapuensis, Bacillus zhangzhouensis, or combinations thereof.

In some examples, the bacterial strains that form spores may be strains of Bacillus, including: Bacillus sp. strain SD-6991; Bacillus sp. strain SD-6992; Bacillus sp. strain NRRL B-50606; Bacillus sp. strain NRRL B-50887; Bacillus pumilus strain NRRL B-50016; Bacillus amyloliquefaciens strain NRRL B-50017; Bacillus amyloliquefaciens strain PTA-7792 (previously classified as Bacillus atrophaeus); Bacillus amyloliquefaciens strain PTA-7543 (previously classified as Bacillus atrophaeus); Bacillus amyloliquefaciens strain NRRL B-50018; Bacillus amyloliquefaciens strain PTA-7541; Bacillus amyloliquefaciens strain PTA-7544; Bacillus amyloliquefaciens strain PTA-7545; Bacillus amyloliquefaciens strain PTA-7546; Bacillus subtilis strain PTA-7547; Bacillus amyloliquefaciens strain PTA-7549; Bacillus amyloliquefaciens strain PTA-7793; Bacillus amyloliquefaciens strain PTA-7790; Bacillus amyloliquefaciens strain PTA-7791; Bacillus subtilis strain NRRL B-50136 (also known as DA-33R, ATCC accession No. 55406); Bacillus amyloliquefaciens strain NRRL B-50141; Bacillus amyloliquefaciens strain NRRL B-50399; Bacillus licheniformis strain NRRL B-50014; Bacillus licheniformis strain NRRL B-50015; Bacillus amyloliquefaciens strain NRRL B-50607; Bacillus subtilisstrain NRRL B-50147 (also known as 300R); Bacillus amyloliquefaciensstrain NRRL B-50150; Bacillus amyloliquefaciens strain NRRL B-50154; Bacillus megaterium PTA-3142; Bacillus amyloliquefaciens strain ATCC accession No. 55405 (also known as 300); Bacillus amyloliquefaciens strain ATCC accession No. 55407 (also known as PMX); Bacillus pumilus NRRL B-50398 (also known as ATCC 700385, PMX-1, and NRRL B-50255); Bacillus cereus ATCC accession No. 700386; Bacillus thuringiensis ATCC accession No. 700387 (all of the above strains are available from Novozymes, Inc., USA); Bacillus amyloliquefaciens FZB24 (e.g., isolates NRRL B-50304 and NRRL B-50349 TAEGRO® from Novozymes), Bacillus subtilis (e.g., isolate NRRL B-21661 in RHAPSODY®, SERENADE® MAX and SERENADE® ASO from Bayer CropScience), Bacillus pumilus (e.g., isolate NRRL B-50349 from Bayer CropScience), Bacillus amyloliquefaciens TrigoCor (also known as "TrigoCor 1448"; e.g., isolate Embrapa Trigo Accession No. 144/88.4Lev, Cornell Accession No.Pma007BR-97, and ATCC accession No. 202152, from Cornell University, USA) and combinations thereof.

In some examples, the bacterial strains that form spores may be strains of Bacillus amyloliquefaciens. For example, the strains may be Bacillus amyloliquefaciens strain PTA-7543 (previously classified as Bacillus atrophaeus), and/or Bacillus amyloliquefaciens strain NRRL B-50154, Bacillus amyloliquefaciens strain PTA-7543 (previously classified as Bacillus atrophaeus), Bacillus amyloliquefaciens strain NRRL B-50154, or from other Bacillus amyloliquefaciens organisms.

The bacterial spores may have an average particle diameter of about 2-50 microns, suitably about 10-45 microns. Bacillus spores are commercially available in blends in aqueous carriers and are insoluble in the aqueous carriers. Other commercially available bacillus spore blends include without limitation Freshen Free™ CAN (10X), available from Novozymes Biologicals, Inc.; Evogen® Renew Plus (10X), available from Genesis Biosciences, Inc.; and Evogen® GT (10X, 20X and 110X), all available from Genesis Biosciences, Inc. In the foregoing list, the parenthetical notations (10X, 20X, and 110X) indicate relative concentrations of the Bacillus spores.

Bacterial spores used in the compositions, methods, and uses disclosed herein may or may not be heat activated. In some examples, the bacterial spores are heat activated. In some examples, the bacterial spores are not heat inactivated. Preferably, the spores used herein are heat activated. Heat activation may comprise heating bacterial spores from room temperature (15- 25°C) to optimal temperature of between 25-120°C, preferably between 40C-100°C, and held the optimal temperature for not more than 2 hours, preferably between 70-80°C for 30 min.

For the methods, compositions and uses disclosed herein, populations of bacterial spores are generally used. In some examples, a population of bacterial spores may include bacterial spores from a single strain of bacterium. Preferably, a population of bacterial spores may include bacterial spores from 2, 3, 4, 5, or more strains of bacteria. Generally, a population of bacterial spores contains a majority of spores and a minority of vegetative cells. In some examples, a population of bacterial spores does not contain vegetative cells. In some examples, a population of bacterial spores may contain less than about 1%, 2%, 3%, 4%, 5%, 6%, 7%, 8%, 9%, 10%, 15%, 20%, 25%, 30%, 40%, or 50% vegetative cells, where the percentage of bacterial spores is calculated as ((vegetative cells/ (spores in population + vegetative cells in population)) $\times$ 100). Generally, populations of bacterial spores used in the disclosed methods, compositions and products are stable (i.e. not undergoing germination), with at least some individual spores in the population capable of germinating.

Populations of bacterial spores used in this disclosure may contain bacterial spores at different concentrations. In various examples, populations of bacterial spores may contain, without limitation, at least $1 \times 10^2$, $5 \times 10^2$, $1 \times 10^3$, $5 \times 10^3$, $1 \times 10^4$, $5 \times 10^4$, $1 \times 10^5$, $5 \times 10^5$, $1 \times 10^6$, $5 \times 10^6$, $1 \times 10^7$, $5 \times 10^7$, $1 \times 10^8$, $5 \times 10^8$, $1 \times 10^9$, $5 \times 10^9$, $1 \times 10^{10}$, $5 \times 10^{10}$, $1 \times 10^{11}$, $5 \times 10^{11}$, $1 \times 10^{12}$, $5 \times 10^{12}$,

l×l0$^{13}$, 5×l0$^{13}$, l×l0$^{14}$, or 5×l0$^{14}$ spores/ml, spores/gram, or spores/cm3.

Detergent composition

The detergent composition is preferably a laundry detergent composition. The composition may be in the form of a solid, a liquid, or a mixture thereof. Preferably the composition is a liquid composition.

A solid can be in the form of free-flowing particulates, compacted solids or a mixture thereof. It should be understood, that a solid may comprise some water, but is essentially free of water. In other words, no water is intentionally added other than what comes from the addition of various raw materials.

In relation to the laundry detergent composition of the present invention, the term 'liquid' encompasses forms such as dispersions, gels, pastes and the like. The liquid composition may also include gases in suitably subdivided form. The term 'liquid laundry detergent composition' refers to any laundry detergent composition comprising a liquid capable of wetting and treating fabric e.g., cleaning clothing in a domestic washing machine. A dispersion for example is a liquid comprising solid or particulate matter contained therein.

The laundry detergent composition may comprise perfume capsules.

Preferably, the laundry detergent composition comprises a non-soap surfactant. The non-soap surfactant is preferably selected from non-soap anionic surfactant, non-ionic surfactant, or a mixture thereof. Preferably, the laundry detergent composition comprises between 10% and 60%, more preferably between 20% and 55% by weight of the laundry detergent composition of the non-soap surfactant.

Preferably, the anionic non-soap surfactant comprises linear alkylbenzene sulphonate, alkyl sulphate, alkoxylated alkyl sulphate, or a mixture thereof. Preferably, the alkoxylated alkyl sulphate is an ethoxylated alkyl sulphate.

Preferably, the laundry detergent composition comprises between 5% and 60%, preferably between 15% and 55%, more preferably between 25% and 50%, most preferably between 30% and 45% by weight of the detergent composition of the non-soap anionic surfactant.

Preferably, the non-soap anionic surfactant comprises linear alkylbenzene sulphonate and alkoxylated alkyl sulphate, wherein the ratio of linear alkylbenzene sulphonate to alkoxylated alkyl sulphate preferably the weight ratio of linear alkylbenzene sulphonate to ethoxylated alkyl sulphate is from 1:10 to 10:1, preferably from 6:1 to 1:6, more preferably from 4:1 to 1:4, even more preferably from 4:1 to 1:1. Alternatively the weight ratio of linear alkylbenzene sulphonate to ethoxylated alkyl sulphate is from 1:2 to 1:4. The alkoxylated alkyl sulphate can be derived from a synthetic alcohol or a natural alcohol, or from a blend thereof, pending the desired average alkyl carbon chain length and average degree of branching. Preferably, the synthetic alcohol is made following the Ziegler process, OXO-process, modified OXO-process, the Fischer Tropsch process, Guerbet process, or a mixture thereof. Preferably, the naturally derived alcohol is derived from natural oils, preferably coconut oil, palm kernel oil, or a mixture thereof.

Preferably, the laundry detergent composition comprises between 0% and 30%, preferably between 1% and 25%, more preferably between 3% and 20%, most preferably between 5% and 20% by weight of the laundry detergent composition of a non-ionic surfactant. Preferably the weight ratio of non-soap anionic surfactant to nonionic surfactant is from 1:2 to 20:1, from 1:1.5 to 15:1, from 1:1 to 10:1, or from 1.5:1 to 5:1. The non-ionic surfactant is preferably selected from alcohol alkoxylate non-ionic surfactant, including naturally derived alcohol, synthetic derived alcohol based alcohol alkoxylate non-ionic surfactants, and mixtures thereof, pending the desired average alkyl carbon chain length and average degree of branching. The alcohol alkoxylate nonionic surfactant can be a primary or a secondary alcohol alkoxylate nonionic surfactant, preferably a primary alcohol alkoxylate nonionic surfactant. Synthetically derived alcohol alkoxylate non-ionic surfactants include Ziegler-synthesized alcohol alkoxylate, an oxo-synthesized alcohol alkoxylate, a modified oxo-process synthesized alcohol alkoxylate, Fischer-Tropsch synthesized alcohol alkoxylates, Guerbet alcohol alkoxylates, alkyl phenol alcohol alkoxylates, or a mixture thereof. The alkoxylation chain can be a mixed alkoxylation chain comprising ethoxy, propoxy and/or butoxy units, or can be a purely ethoxylated alkyl chain, preferably a purely ethoxylated alkyl chain.

Preferably, the laundry preferably liquid laundry detergent composition comprises between 1% and 20%, more preferably between 2% and 15%, even more preferably between 3% and 10%, most preferably between 4% and 8% by weight of the laundry detergent composition of soap, preferably a fatty acid salt, more preferably an amine neutralized fatty acid salt, wherein preferably the amine is an alkanolamine more preferably selected from monoethanolamine, diethanolamine, triethanolamine or a mixture thereof, more preferably monoethanolamine.

Preferably, the laundry detergent composition comprises a non-aqueous solvent, preferably wherein the non-aqueous solvent is selected from ethanol, 1,2-propanediol, dipropylene glycol, tripropyleneglycol, glycerol, sorbitol, ethyleneglycol, polyethylene glycol, polypropylene glycol, or a mixture thereof, preferably wherein the polypropyleneglycol has a molecular weight of 400. Preferably the liquid laundry detergent composition comprises between 10% and 40%, preferably between 15% and 30% by weight of the liquid laundry detergent composition of the non-aqueous solvent. Without wishing to be bound by theory the non-aqueous solvents ensure appropriate levels of film plasticization so the film is not too brittle and not too 'floppy'. Without wishing to be bound by theory, having the correct degree of plasticization will also facilitate film dissolution when exposed to water during the wash process.

Preferably, the liquid laundry detergent composition comprises between 1% and 20%, preferably between 5% and 15% by weight of the liquid laundry detergent composition of water.

Preferably, the laundry detergent composition comprises an ingredient selected from the list comprising cationic polymers, polyester terephthalate polymers, amphiphilic graft copolymers, alkoxylated preferably ethoxylated polyethyleneimine polymers, carboxymethylcellulose, enzymes, bleach, or a mixture thereof.

Preferably, the laundry detergent composition comprises perfume, preferably from 0.01% to 5%, more preferably from 0.05% to 3% of perfume by weight of the detergent composition. The perfume comprises one or more, preferably two or more, perfume raw materials. The term "perfume raw material" (or "PRM") as used herein refers to compounds having a molecular weight of at least about 100 g/mol and which are useful in imparting an odor, fragrance, essence, or scent, either alone or with other perfume raw materials. Preferably, the perfume of the detergent of the composition comprises alcohols, ketones, aldehydes, esters, ethers, nitrites and alkenes, such as terpene.

The PRMs may be characterized by their boiling points (B.P.) measured at the normal pressure (760 mm Hg), and their octanol/water partitioning coefficient (P), which may be described in terms of logP, determined according to the test method described in Test methods section in page 23 of WO2022/081781. Based on these characteristics, the PRMs may be categorized as Quadrant I, Quadrant II, Quadrant III, or Quadrant IV perfumes, as described in more detail below. A perfume having a variety of PRMs from different quadrants may be desirable, for example, to provide fragrance benefits at different touchpoints during normal usage.

Perfume raw materials having a boiling point B.P. lower than about 250°C and a logP lower than about 3 are known as Quadrant I perfume raw materials. Quadrant 1 perfume raw materials are preferably limited to less than 30% of the perfume composition. Perfume raw materials having a B.P. of greater than about 250°C and a logP of greater than about 3 are known as Quadrant IV perfume raw materials, perfume raw materials having a B.P. of greater than about 250°C and a logP lower than about 3 are known as Quadrant II perfume raw materials, perfume raw materials having a B.P. lower than about 250°C and a logP greater than about 3 are known as a Quadrant III perfume raw materials.

Preferably, the perfume comprises a mixture of at least 3, or even at least 5, or at least 7 perfume raw materials. The perfume may comprise at least 10 or at least 15 perfume raw materials. A mixture of perfume raw materials may provide more complex and desirable aesthetics, and/or better perfume performance or longevity, for example at a variety of touchpoints. However, it may be desirable to limit the number of perfume raw materials in the perfume to reduce or limit formulation complexity and/or cost.

The perfume may comprise at least one perfume raw material that is naturally derived. Such components may be desirable for sustainability/environmental reasons. Naturally derived perfume raw materials may include natural extracts or essences, which may contain a mixture of PRMs. Such natural extracts or essences may include orange oil, lemon oil, rose extract, lavender, musk, patchouli, balsamic essence, sandalwood oil, pine oil, cedar, and the like.

The laundry detergent composition may comprise free perfume and/or encapsulated perfume.

The laundry detergent composition may comprise an adjunct ingredient, wherein the adjunct ingredient is selected from hueing dyes, aesthetic dyes, builders preferably citric acid, chelants, cleaning polymers, dispersants, dye transfer inhibitor polymers, fluorescent whitening agent, opacifier, antifoam, preservatives, anti-oxidants, or a mixture thereof. Preferably the chelant is selected from aminocarboxylate chelants, aminophosphonate chelants, or a mixture thereof.

Preferably, the laundry detergent composition has a pH between 6 and 10, more preferably between 6.5 and 8.9, most preferably between 7 and 8, wherein the pH of the laundry detergent composition is measured as a 10% solution in demineralized water at 20°C.

The liquid laundry detergent composition may be Newtonian or non-Newtonian. Preferably, the liquid laundry detergent composition is non-Newtonian. Without wishing to be bound by theory, a non-Newtonian liquid has properties that differ from those of a Newtonian liquid, more specifically, the viscosity of non-Newtonian liquids is dependent on shear rate, while a Newtonian liquid has a constant viscosity independent of the applied shear rate. The decreased viscosity upon shear application for non-Newtonian liquids is thought to further facilitate liquid detergent dissolution. The liquid laundry detergent composition described herein can have any suitable viscosity depending on factors such as formulated ingredients and purpose of the composition. When Newtonian the composition may have a viscosity value, at a shear rate of 20s-1 and a temperature of 20°C, of 100 to 3,000 cP, alternatively 200 to 2,000 cP, alternatively 300 to 1,000 cP, following the method described herein. When non-Newtonian, the composition may have a high shear viscosity value, at a shear rate of 20s-1 and a temperature of 20°C, of 100 to 3,000 cP, alternatively 300 to 2,000 cP, alternatively 500 to 1,000 cP, and a low shear viscosity value, at a shear rate of 1 s-1 and a temperature of 20°C, of 500 to 100,000 cP, alternatively 1000 to 10,000 cP, alternatively 1,300 to 5,000 cP, following the method described herein. Methods to measure viscosity are known in the art. According to the present disclosure, viscosity measurements are carried out using a rotational rheometer e.g. TA instruments AR550. The instrument includes a 40mm 2° or 1 ° cone fixture with a gap of around 50-60μm for isotropic liquids, or a 40mm flat steel plate with a gap of 1000 μm for particles containing liquids. The measurement is carried out using a flow procedure that contains a conditioning step, a peak hold and a continuous ramp step. The conditioning step involves the setting of the measurement temperature at 20°C, a pre-shear of 10 seconds at a shear rate of 10s1, and an equilibration of 60 seconds at the selected temperature. The peak hold involves applying a shear rate of 0.05s1 at 20°C for 3min with sampling every 10s. The continuous ramp step is performed at a shear rate from 0.1 to 1200s1 for 3min at 20°C to obtain the full flow profile.

## Method of making the water-soluble unit dose article

Those skilled in the art will be aware of known techniques and methods to make the laundry detergent composition and the water-soluble unit dose article.

The method of making an article according to the invention comprises the steps of:

a) forming a film comprising bacterial spores; and

b) encapsulating the detergent composition in the water-soluble film.

A preferred way of incorporating bacterial spores into the water-soluble film includes creation of a premix solution comprising the water-soluble resin, the bacterial spores and other adjunct film materials as described herein and then casting this solution to create of a water-solution film comprising the bacterial spores dispersed therein.

## Method of use

A further aspect of the present invention is a method of laundering fabrics comprising the steps of diluting preferably between 200 and 3000 fold, preferably between 300 and 2000 fold, the water-soluble unit dose article according to the present invention with water to make a wash liquor, contacting fabrics to be treated with the wash liquor.

The wash liquor may comprise water of any hardness preferably varying between 0 gpg to 40gpg.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

Every document cited herein, including any cross referenced or related patent or application and any patent application or patent to which this application claims priority or benefit thereof, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

## Examples

The following example compares the colour stability of soluble unit dose (SUD) detergents where *Bacillus* spores have been added to the liquid detergent versus addition to the film used to encapsulate the liquid detergent. The results show that delivery of *Bacillus* spores to the film achieves a significant improvement in colour stability compared to the same material added directly to the liquid detergent.

## Detergent and stability test method

The SUD liquid detergent was prepared with the following composition:

|  | % active in formula |
|---|---|
| Nonionic surfactant | 10 |
| Linear alkylbenzene sulfonate[1] | 27 |
| Alcohol ether sulfate (C12-14AE3S)[2] | 1.5 |

(continued)

|  | % active in formula |
|---|---|
| Ethoxylated Polyethyleneimine (PEI600E020)[3] | 4 |
| PEG vinyl acetate co polymer[4] | 2.5 |
| C12-18 Fatty acid[5] | 4.3 |
| Citric acid[6] | 1.4 |
| Tetrasodium N,N-Dicarboxymethyl glutamate (GLDA) | 0.9 |
| Monoethanolamine | 7.2 |
| Glycerin[7] | 2.6 |
| Fluorescent brightener 49 | 1.1 |
| Perfume | 2 |
| Preservative | 0.4 |
| Structurant (Hydrogenated Castor Oil) | 0.1 |
| Water | 2.3 |
| Propylene Glycol[8] | balance |

1 Linear alkylbenzene sulfonate, made using HF process, alternatively it may be made using AlCL3 (Friedel Craft) or DETAL processes, or being derived from an $\alpha$-olefin comprising more than 50% by weight thereof of C12 $\alpha$-olefin or more than 75% by weight thereof of C12 $\alpha$-olefin or consisting essentially of C12 $\alpha$-olefin
2 C1214AE3S, derived from natural oil e.g. coconut oil, alternatively it may be derived from palm kernel oil
3 Lutensol FP620 ex BASF - ethoxylated polyethyleneimine (PEI600 EO20)
4 polyethylene glycol graft polymer comprising a polyethylene glycol backbone (Pluriol E6000) and hydrophobic vinyl acetate side chains, comprising 40% by weight of the polymer system of a polyethylene glycol backbone polymer and 60% by weight of the polymer system of the grafted vinyl acetate side chains
5 C12-18 fatty acid derived from palm kernel oil, alternatively it may be made from coconut oil
6 synthetic citric acid, alternatively it may be made from a natural source
7 synthetic glycerin, alternatively it may be made from a natural source
8 synthetic propylene glycol, alternatively it may be made from a natural source

Perfume composition:

| Perfume raw material | Level (%) |
|---|---|
| ALLYL AMYL GLYCOLATE | 1.500 |
| AMBRETTOLIDE | 2.000 |
| AMBRONAT | 0.600 |
| AMYL PROPIONATE | 1.500 |
| ANISYL ACETATE | 1.000 |
| BENZYL ACETATE | 6.000 |
| BETA GAMMA HEXENOL | 1.000 |
| BETA NAPHTHOL METHYL ETHER | 2.000 |
| CINNAMIC ALDEHYDE | 0.400 |
| CITRAL | 2.000 |
| CITRONELLYL NITRILE | 2.000 |
| CLONAL | 0.400 |

(continued)

| Perfume raw material | Level (%) |
|---|---|
| CORANOL | 5.000 |
| CYCLOHEXYL SALICYLATE | 5.100 |
| DELTA DAMASCONE | 1.500 |
| DELTA OCTALACTONE FCC | 0.600 |
| DIPENTENE | 7.000 |
| ETHYL BUTYRATE | 0.400 |
| ETHYL MALTOL | 0.600 |
| ETHYL-2-METHYL BUTYRATE | 1.500 |
| ETHYLENE BRASSYLATE | 5.000 |
| EUCALYPTOL | 3.000 |
| EUGENOL | 0.600 |
| FLORHYDRAL | 1.500 |
| GERANIOL | 6.000 |
| HELIOTROPIN | 1.500 |
| HEXYL ACETATE | 2.000 |
| IONONE BETA | 4.000 |
| ISO BORNYL ACETATE | 4.000 |
| ISO E SUPER OR WOOD | 4.000 |
| LACTOJASMON | 0.400 |
| LINALYL ACETATE | 5.000 |
| METHYL BETA-NAPHTHYL KETONE | 1.500 |
| METHYL DIHYDRO JASMONATE | 3.900 |
| METHYL NONYL ACETALDEHYDE | 1.500 |
| METHYL SALICYLATE USP | 0.600 |
| PEONILE | 3.000 |
| PHENYL ETHYL ALCOHOL | 5.000 |
| PHENYL ETHYL DIMETHYL CARBINOL | 2.000 |
| PRENYL ACETATE | 1.000 |
| STRAWBERIFF | 0.400 |
| VANILLIN | 2.000 |

Polyvinyl alcohol film solution was prepared to give a final composition of 64 wt.% Mowiol 18-88 solution (81365, Sigma Aldrich), 20 wt% glycerol (Verbio Vereinigte BioEnergie AG, Leipzig) and 10 wt % D-sorbitol (S1876, Sigma Aldrich). Solutions were mixed at elevated temperature (100°C) until the PVA was fully dissolved. The solution was then mixed at room temperature until equilibrated. *Bacillus* spore premix (Genesis Biosciences, Cardiff, UK) was added to the PVA solutions at a level to achieve a final activity of $1.9 \times 10^8$ cfu/g dry PVA.

The solution was cast overnight on a plastic casting board to give a PVA sheet of uniform thickness.

22g of the SUD liquid detergent was weighed out into $3 \times 60$ml clean glass jars. To the reference (A) 0.7g of reference nil spore PVA film (MonoSol, Merrillville, IN, USA) was added. To the second jar (B) 0.7g of the *Bacillus* spore PVA film was added. Both were mixed to ensure contact of the detergent with the film. To the third jar (C) *Bacillus* spore powder (Genesis Biosciences, Cardiff, UK) was added to the liquid detergent to achieve an equivalent activity of spores as was delivered via the PVA film in jar B ($1.33 \times 10^8$ cfu) and mixed thoroughly. 0.7g of reference nil spore PVA film was then added to jar C and mixed to ensure contact of the detergent with the film.

| Test leg | |
|---|---|
| A | Reference - SUD liquid detergent + PVA |
| B | SUD liquid detergent + *Bacillus* spore PVA |
| C | SUD liquid detergent + *Bacillus* spores + PVA |

The jars containing the detergent and film mixes were then placed in an oven at 35°C.

After three weeks 2g of each detergent mixture stored at 35°C was pipetted into a 20ml glass vial. L*a*b* measurements in D65 were taken using a DigiEye (VeriVide Ltd, Leicester, UK) at shutter speed 1/2, radius 180 which was calibrated before use, by imaging down into the open glass vial. Delta E values were calculated from the L*a*b* values of the reference (nil spore) vs the test legs, using the following calculation:

$$\Delta E_{ab}^* = \sqrt{(L_2^* - L_1^*)^2 + (a_2^* - a_1^*)^2 + (b_2^* - b_1^*)^2}$$

| 35°C storage, 3 weeks | L* value (Stdev) | a* value (Stdev) | b* value (Stdev) | Δb* value vs. ref | Delta E* vs. ref (Stdev) |
|---|---|---|---|---|---|
| A - Reference - SUD liquid detergent + PVA | 82.16 (0.47) | -1.11 (0.14) | 13.53 C* (0.29) | - | - |
| B - SUD liquid detergent + *Bacillus* spore embedded in PVA | 81.85 (0.59) | -1.06 (0.18) | 12.72 C* (0.32) | -0.82 C* | 1.03 (0.57) |
| C - SUD liquid detergent containing *Bacillus* spores + PVA | 80.19 (0.26) | -0.82 (0.09) | 16.94 **AB*** (0.48) | **+3.31 AB*** | **3.97** (0.54) |
| *Letters in bold indicate significant differences in b* values as determined by Tukey-Kramer HSD (p-value =<0.0001). | | | | | |

The SUD liquid detergent in jar C comprising *Bacillus* spores and PVA film showed a significant yellowing, measured as a significant increase in b* value (more positive = more yellow) after three weeks storage at 35°C. This correlates with a higher Delta E* value of 3.97 which is highly noticeable to the eye. However, this yellowing of the liquid detergent did not occur when the same quantity of *Bacillus* spores was incorporated into the PVA film. The results suggest that *Bacillus* spores cause a colour stability issue in SUD liquid detergents but this can be mitigated by delivering the *Bacillus* spores into the film.

## Claims

1.   A water-soluble unit-dose detergent article comprising a water-soluble film and a detergent composition, wherein the water-soluble film comprises bacterial spores.

2.   An article according to claim 1 wherein the bacterial spores comprise *Bacillus* spores.

3.   An article according to the preceding claim wherein the *Bacillus* spores comprise bacteria selected from the group consisting of *Bacillus subtilis, Bacillus amyloliquefaciens, Bacillus licheniformis, Bacillus megaterium, Bacillus pumilus, Bacillus thuringiensis, Bacillus coagulans,* and mixtures thereof.

4.   An article according to any of the preceding claims wherein the film comprises from $1 \times 10^3$ to $1 \times 10^{11}$ CFU/g of bacterial spores by weight of the film.

5.   An article according to any of the preceding claims wherein the film comprises a water-soluble resin comprising polyvinyl alcohol.

6.   An article according to any of claims 1 to 4 wherein the film comprises a water-soluble resin comprising a material selected from the group consisting of protein, polysaccharide, and a mixture thereof.

7. An article according to the preceding claim wherein the film comprises a water-soluble resin comprising casein.

8. An article according to claim 6 wherein the film comprises a water-soluble resin comprising pectin.

9. An article according to claim 6 wherein the film comprises a water-soluble resin comprising carrageenan.

10. An article according to any of the preceding claims wherein the film further comprises an additive selected from the group consisting of plasticizers, plasticizer compatibilizers, lubricants, release agents, fillers, extenders, cross-linking agents, antiblocking agents, antioxidants, detackifying agents, antifoams, nanoparticles, bleaching agents, surfactants, bittering agents, and combinations thereof.

11. An article according to any of the preceding claims wherein the film is coated with a powder.

12. An article according to any of the preceding claims wherein the detergent composition comprises between 5% and 60% by weight of the detergent composition of a non-soap anionic surfactant.

13. An article according to any of the preceding claims wherein the detergent composition comprises between 2.5% and 30% by weight of the detergent composition of a non-ionic surfactant.

14. An article according to any of the preceding claims wherein the detergent composition comprises between 1% and 15% by weight of the detergent composition of water.

15. An article according to any of the preceding claims, wherein the detergent composition further comprises an alkanolamine selected from the group comprising of monoethanolamine, diethanolamine, triethanolamine, and a mixture thereof, preferably monoethanolamine.

16. An article according to any of the preceding claims, wherein the detergent composition further comprises a perfume.

17. An article according to any of the preceding claims wherein the detergent composition comprises an adjunct comprising one or more of: soap surfactants, peroxy compounds, bleach activators, anti-redeposition agents, neutralizers, optical brighteners, foam inhibitors, chelators, builders, enzymes, hueing dyes, bittering agents, dye transfer inhibitors, soil release agents, water softeners, electrolytes, pH regulators, anti-graying agents, anti-crease components, bleach agents, reducing agents, colorants, perfume capsules, processing aids, and mixtures thereof.

18. A method of making an article according to any preceding claim, wherein said method comprises the steps of:

    a) forming a film comprising bacterial spores; and
    b) encapsulating the detergent composition in the water-soluble film.

19. A method of laundering a fabric comprising the step of contacting the fabric with an aqueous solution comprising the article according to any of claims 1 to 15.

Europäisches
Patentamt

European
Patent Office

Office européen
des brevets

# EUROPEAN SEARCH REPORT

**Application Number**

EP 24 18 8554

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 342 846 A1 (GRUPA INCO S A [PL]) 4 July 2018 (2018-07-04) | 1-5, 10-17,19 | INV. C11D3/37 |
| Y | * paragraph [1;5;45;46;56;60;93]; claim 1; figures 1-3; example 5; table 7 * | 6-9,18 | C11D3/38 C11D17/04 |
| | ----- | | |
| Y | US 2024/076587 A1 (COURCHAY FLORENCE CATHERINE [BE] ET AL) 7 March 2024 (2024-03-07) * claim 1 * | 6,7 | |
| | ----- | | |
| Y | US 2003/148914 A1 (DASQUE BRUNO MATTHIEU [BE] ET AL) 7 August 2003 (2003-08-07) * paragraph [0070] * | 8 | |
| | ----- | | |
| Y | US 2021/238514 A1 (JANSSEN FRANK [DE] ET AL) 5 August 2021 (2021-08-05) * paragraph [50;51] * | 9 | |
| | ----- | | |
| Y | US 2017/259976 A1 (LEE DAVID M [US] ET AL) 14 September 2017 (2017-09-14) * paragraph [77;93;127;128;168]; claims 1,13 * | 18 | |
| | ----- | | |

**TECHNICAL FIELDS SEARCHED (IPC)**

C11D

The present search report has been drawn up for all claims

| Place of search | Date of completion of the search | Examiner |
|---|---|---|
| The Hague | 6 January 2025 | Douelle, Frédéric |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 18 8554

This annex lists the patent family members relating to the patent documents cited in the above-mentioned European search report.
The members are as contained in the European Patent Office EDP file on
The European Patent Office is in no way liable for these particulars which are merely given for the purpose of information.

06-01-2025

| Patent document cited in search report | | | Publication date | Patent family member(s) | | | Publication date |
|---|---|---|---|---|---|---|---|
| EP 3342846 | A1 | | 04-07-2018 | NONE | | | |
| US 2024076587 | A1 | | 07-03-2024 | CN | 117460813 | A | 26-01-2024 |
| | | | | EP | 4105308 | A1 | 21-12-2022 |
| | | | | JP | 2024519017 | A | 08-05-2024 |
| | | | | US | 2024076587 | A1 | 07-03-2024 |
| | | | | WO | 2022266026 | A1 | 22-12-2022 |
| US 2003148914 | A1 | | 07-08-2003 | AT | E362518 | T1 | 15-06-2007 |
| | | | | CA | 2460060 | A1 | 08-05-2003 |
| | | | | DE | 60220178 | T2 | 10-01-2008 |
| | | | | EP | 1446472 | A1 | 18-08-2004 |
| | | | | ES | 2287341 | T3 | 16-12-2007 |
| | | | | US | 2003148914 | A1 | 07-08-2003 |
| | | | | WO | 03038027 | A1 | 08-05-2003 |
| US 2021238514 | A1 | | 05-08-2021 | EP | 3867342 | A1 | 25-08-2021 |
| | | | | US | 2021238514 | A1 | 05-08-2021 |
| | | | | WO | 2020079168 | A1 | 23-04-2020 |
| US 2017259976 | A1 | | 14-09-2017 | CN | 107108919 | A | 29-08-2017 |
| | | | | EP | 3201100 | A2 | 09-08-2017 |
| | | | | JP | 6720150 | B2 | 08-07-2020 |
| | | | | JP | 2017537989 | A | 21-12-2017 |
| | | | | KR | 20170065618 | A | 13-06-2017 |
| | | | | TW | 201630989 | A | 01-09-2016 |
| | | | | US | 2017259976 | A1 | 14-09-2017 |
| | | | | US | 2022017276 | A1 | 20-01-2022 |
| | | | | US | 2024150099 | A1 | 09-05-2024 |
| | | | | WO | 2016054528 | A2 | 07-04-2016 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2022081781 A